Europäisches Patentamt

**European Patent Office**

Office européen des brevets

(11) Publication number: **0 412 554 B1**

# EUROPEAN PATENT SPECIFICATION

(45) Date of publication of patent specification: **02.11.94**  (51) Int. Cl.5: **A61K 9/22**, A61K 9/20

(21) Application number: **90115389.0**

(22) Date of filing: **10.08.90**

The file contains technical information submitted after the application was filed and not included in this specification

(54) **Sustained-release preparation for administration into brain.**

(30) Priority: **10.08.89 JP 208484/89**

(43) Date of publication of application:
**13.02.91 Bulletin 91/07**

(45) Publication of the grant of the patent:
**02.11.94 Bulletin 94/44**

(84) Designated Contracting States:
**AT BE CH DE DK ES FR GB GR IT LI LU NL SE**

(56) References cited:
**EP-A- 0 139 286**
**EP-A- 0 230 647**
**EP-A- 0 251 631**

**American Stedman's Medical Dictionary page 1548**

**Dorlands Medical Dictionary page 1357**

(73) Proprietor: **SUMITOMO PHARMACEUTICALS COMPANY, LIMITED**
**2-8, Dosho-machi 2-Chome,**
**Chuo-ku**
**Osaka-shi Osaka-fu (JP)**

Proprietor: **Koken Company Limited**

5-18, Shimo-Ochiai 3-chome
Shinjuku-ku Tokyo (JP)

(72) Inventor: **Hayakawa, Toru**
**No. 1-2-10-107, Mikageyamate-cho**
**Higashinada-ku, Kobe-shi, Hyogo-ken (JP)**
Inventor: **Yoshimine, Toshiki**
**No. 3-7, Takezono-cho**
**Ashiya-shi, Hyogo-ken (JP)**
Inventor: **Fujioka, Keiji**
**No. 1-27-5-206, Tsukaguchi-cho**
**Amagasaki-shi, Hyogo-ken (JP)**
Inventor: **Takada, Yoshihiro**
**No. 4-37-1-813, Senriyama-Nishi**
**Suita-shi, Osaka-fu (JP)**
Inventor: **Sasaki, Yoshio**
**No. 1-19-28, Tsunoe-cho**
**Takatsuki-shi, Osaka-fu (JP)**
Inventor: **Irie, Tsunemasa**
**No. 2-6-12, Mukovama**
**Takatzuka-shi, Hyogo-ken (JP)**
Inventor: **Fukushima, Nobuyuki**
**No. 2-11-7-406, Sone-Higashi-cho**
**Toyonaka-shi, Osaka-fu (JP)**

(74) Representative: **VOSSIUS & PARTNER**
**Postfach 86 07 67**
**D-81634 München (DE)**

**Description**

The present invention relates to a sustained-release preparation suitable for administering a pharmaceutically active substance for the treatment of cerebral diseases into the brain which comprises the active substance incorporated into a biodegradable carrier,

In EP-A 0251631 interleukin 2 containing composition is discribed wherein a controlled-release material may be used for inter cranial implantation.

The invention provides a sustained-release preparation of a pharmaceutically active substance for the treatment of cerebral diseases wherein the active substance is incorporated into a biodegradable carrier selected from collagen, gelatin and a mixture thereof, which can release with reasonable duration such an active substance that is hardly delivered into the brain when it is administered by a conventional administration method, and thereby can exhibit the activity thereof for a long time. In the present invention, the term of "brain" means both "brain parenchyma" and "cerebrospinal fluid (CSF) space". The "brain parenchyma" includes cerebrum, brain stem and cerebellum.

The blood vessels in the brain parenchyma have peculiar characteristics, by which it hinders the random transport into the brain of components in blood or a drug administered or absorbed into blood. The inside of blood capillary in brain parenchyma is covered with endothelial cells having low material permeability, and these endothelial cells are bound together by tight junctions so that many substances cannot pass through them. This barrier in the cerebrovascular vessels between blood and brain is called blood-brain barrier (BBB), which becomes a serious barrier for drug-transport into the brain when it is needed.

Hitherto, there have been many attempts to transfer a drug effectively into the brain through BBB. For instance, S. I. Rapoport et al. reported that when a drug is administered simultaneously with a hypertonic solution of mannitol and the like, BBB is loosened so that the vascular permeability of the drug is increased [Cf: Federation Proceedings, 43 (2), 214 (1984)]. There have also been attempts to develop a drug that passes through BBB, for example, by increasing the lipophilicity of the drug because some lipophilic materials are more easily transported into brain by diffusion through cerebrovascular walls, or by encapsulating a drug within liposome. In these methods, however, the drug is systemically distributed and cannot be selectively delivered into the brain, and hence, it is difficult to transport a therapeutically effective amount of the drug into the brain. As a result, a large quantity of the drug needs to be administered.

Japanese Patent Publication (KOHYO) No. 500901/1989, which is a national publication of Japanese version of PCT/US87/01699 (WO88/00834), discloses a method for transferring a drug physiologically through BBB by binding the drug to a peptide which can pass through BBB by receptor-mediated transcytosis. However, in the above method, it is difficult to transfer a therapeutically effective amount of a drug into the brain when administered by a practically employed administration method such as intravenous injection.

Furthermore, Ommaya reservoir has been invented as the most sure method to deliver a drug into the brain, wherein the drug is delivered into the brain through a catheter implanted intraventricularly. Although it is possible to administer a solution of a drug into the brain with Ommaya reservoir, it does not have a function to deliver continuously a solution of a drug, and hence, its application is limited to intermittent administration of a drug. On the other hand, an implanted infusion pump is used for continuous administration for a long period of time. The said apparatus is, however, expensive and an infection at the catheter inserted site or leak of cerebral spinal fluid tends to occur, which causes a serious problem. The operation thereof dependes on the operator's technique so that there are problems in reliability thereof. Therefore, this method is not widely used in the field of the treatment of cerebral diseases.

On the other hand, there have also been some attempts for local administration into the brain by improvements of dosage form in the field of the treatment of cerebral tumors. Kaetsu et al. reported a sustained-release preparation of an anticancer agent using a nonbiodegradable vinyl copolymer [Cf: Jinko Zoki (Artificial Organ), 15(1), 214 - 217 (1986)]. For instance, they prepared a vinyl copolymer containing an anticancer agent, nimustine hydrochloride (ACNU) and examined the extent of tissue necrosis after administration thereof into the brain. This reference indicates that although necrotic action is different depending on the anticancer agents, the extent to be effected thereby, that is, the extent of distribution of a drug is comparatively limited, and hence, the therapeutic effect is hardly expected to cover the whole lesion.

The pharmaceutically active substance for the treatment of cerebral diseases used in the present invention are substances which are expected to exhibit the effects thereof in partial or whole brain parenchyma and in CSF space, and they have difficulties to pass through BBB and exhibit the effect thereof in the brain by conventional administration methods, and they can hardly diffuse within the brain and cannot be distributed throughout the whole brain even when they are directly administered into the brain. The above tendency is more apparent in substances with higher molecular weight. Besides, since the half-life of a macromolecular substance is short in the living body, the duration time of action thereof is short in the brain even though it is transported into the brain by any of the methods mentioned above, and hence, it needs to be administered frequently and repeatedly in order to increase the therapeutic effects thereof. Therefore, the administration methods into the brain are not widely used for the practical treatment of cerebral diseases.

From many reasons described above, it has been desired to develop a method for administering a therapeutically effective amount of active substances for the treatment of cerebral diseases which can exhibit the activity in the brain with reasonably long duration.

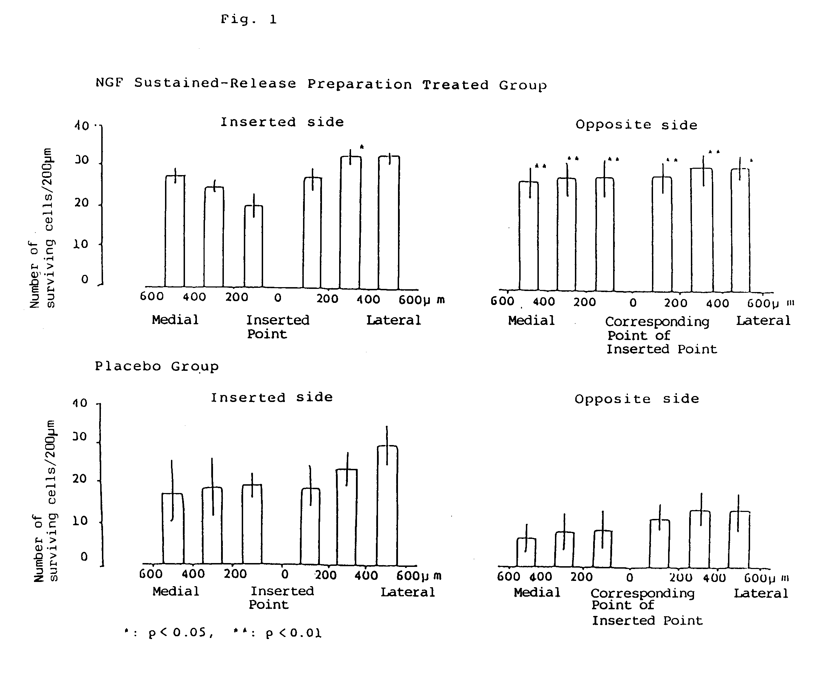

Fig. 1 shows experimental data of the effects of NGF sustained-release preparation of the present invention on the necrosis of hippocampal CAI pyramidal cells of Mongolian gerbil when it was inserted into the hippocampus. The number of surviving cells in the treated group with the NGF sustained-release preparation was significantly larger than that in the placebo preparation group, within the region of 200 - 400 $\mu$m remote from the inserted site and in all the regions in the opposite part.

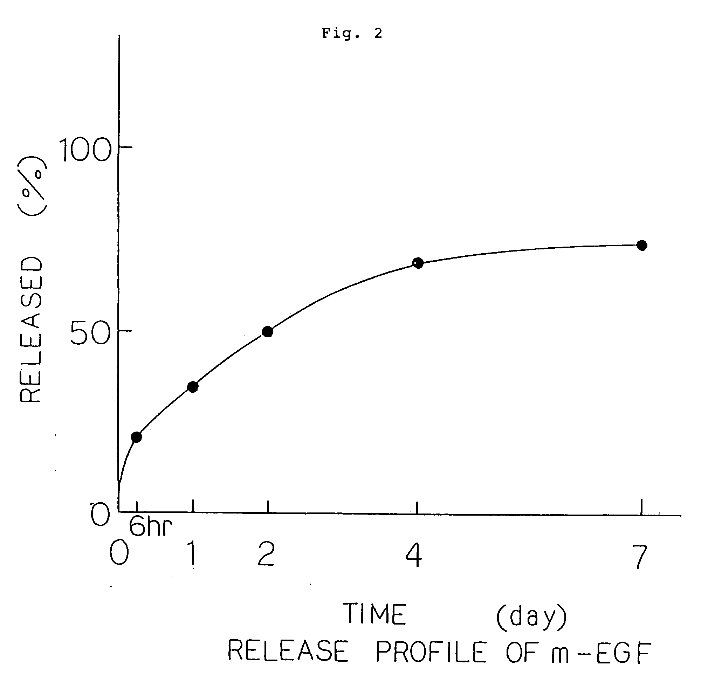

Fig. 2 shows experimental results in the release test (in vitro) of mouse epidermal growth factor (m-EGF) from the m-EGF sustained-release preparation of the present invention, which shows that the sustained-release preparation of the present invention could release the m-EGF with good duration for a long period of time.

The technical problem underlying the present invention is to provide pharmaceutical preparations for the treatment of cerebral diseases which allow the continuous and diffuse distribution of an active substance thereof into the brain and provide a long-lasting activity.

The solution to this technical problem is achieved by providing the pharmaceutical preparations of the present invention which are sustained release preparations and contain the pharmaceutically active substance for the treatment of cerebral diseases incorporated into a biodegradable carrier.

An object of the invention is to provide a sustained-release preparation suitable for administering a pharmaceutically active substance for the treatment of cerebral diseases comprising the active substance incorporated into a specific biodegradable carrier, which can release the active substance and exhibit the activity thereof in the brain with long duration. Another object of the invention is to provide a method for directly administering a pharmaceutically active substance for the treatment of cerebral disease into the brain which is effective for releasing the active substance in the brain without being affected by BBB, and for exhibiting the activity with long duration in the brain. A further object of the invention is to provide use of a pharmaceutically active substance for the treatment of cerebral diseases in the dosage form comprising the active substance incorporated into a specific carrier.

The cerebral diseases to be treated by the present invention are, for example, degenerative disorders of the brain, cerebrovascular dementia or cerebral tumors. The neurodegenerative disorder is a disease wherein nerve cells are degenerated or denervated, for example, Alzheimer's disease, senile dementia of Alzheimer type, amyotrophic lateral selerosis, Parkinson's disease or Pick's disease.

The active substances for the treatment of cerebral diseases used in the present invention are, for example, brain peptides, and a derivative, an antibody, an agonist and an antagonist thereof.

The brain peptides include bioactive substances such as neurotrophic factors, cell growth factors, neurotransmitter-related compounds, immuno-stimulators, or tumor necrosis factor.

The neurotrophic factors are a bioactive substance which influences neuronal survival, differentiation, induction of enzymes and chemotaxis, for example, nerve growth factor (NGF), brain derived neurotrophic factor (BDNF), ciliary neurotrophic factor (CNTF), hippocampus-derived neurotrophic factor, NT-3. The hippocampus-derived neurotrophic factor is, for example, hippocampus factor.

It is known that NGF, which is one of the neurotrophic factors, exists within the central nervous system and acts thereon, and hence, it has been expected to be useful for the treatment of neurodegenerative disorders such as Alzheimer's disease. NGF cannot be distributed in the brain when administered intravenously or orally. Even though NGF is administered intracerebrally or intraventricularly, the duration of action thereof is short. Therefore, NGF needs to be administered frequently and repeatedly in order to increase the therapeutic effect thereof in human, which is a complicated procedure for clinical use and not practical. In the above circumstances, it is desired to develop a simple and effective administration method of NGF.

Hippocampus-derived neurotrophic factor, one of the neurotrophic factors, exists within the hippocampus, and includes various factors such as hippocampus factor, [Cf: Ojika et al., Proc. Natl. Acad. Sci. USA, 81, 2567 - 2571 (1984)].

The cell growth factors are those which enhance the growth, hypertrophy, division and proliferation of cells in vivo and/or in vitro. The cell growth factors include, for example, epidermal growth factor (EGF), fibroblast growth factor (FGF), platelet-derived growth factor (PDGF), endothelial cell growth factor, transforming growth factor (TGF), insulin-like growth factors I and II (IGF-I, II), erythropoietin or thrombopoietin. The epidermal growth factor, acidic fibroblast growth factor and basic fibroblast growth factor are known to have a neurotrophic effect, and therefore attention is given to the therapeutic application thereof into the brain.

There are various peptides which are known as neurotransmitter-related compounds. The peptide hormones relating to memory are, for example, vasopressin, $\beta$-endorphin, enkepharin, oxytocin or cholecystokinin, Since DeWied has firstly reported that vasopressin participates in learning and memory [Cf: Int. J. Neuropharmaco. 14, 157 - 167 (1965)], there has been studied a clinical use of vasopressin in the treatment of memory disorder.

The immuno-stimulators include, for example, interleukin and interferon, Interferon is a glycoprotein with a molecular weight of 21,000 - 24,000, and has three types such as $\alpha$, $\beta$, $\gamma$ thereof. It is known that interferon has antiviral activity and anticancer activity.

Interleukin is one of the lymphokines produced by macrophages, and enhances the activation of the growth of T-cell and B-cell. Lindholm et al. reported that interleukin enhances the synthesis of NGF by neurons. Interleukin has been recognized to be a possible drug for neurodegenerative disorders with an effect of enhancement of a neurotrophic effect of NGF [Cf: Lindholm, D. et al., Nature 330, 658 - 659 (1987)].

Highly purified tumor necrosis factor (TNF) has direct cytotoxic activity on all cancer cells examined in vitro. TNF shows excellent curative effects against all types of murine and human tumors tested in vivo [Cf: Carswell, F.A. et al., Proc. Natl. Acad. Sci. U.S.A. 72, 3666 - 3670 (1975)]. Human TNF is purified and has a molecular weight of approximately 17,000, an isoelectric point 5.3 [Cf: Bharat B. Aggarwal et al., J. Biol. Chem. 260(4), 2345 - 2354 (1984)].

The derivatives or agonists of the brain peptides above having the same bioactivities may be used in the present invention. Further, antagonists or antibodies thereof having an inhibitory effect on the bioactivities of the above brain peptides may also be used in the present invention.

The present invention is especially useful for the administration of high molecular weight substances which can hardly pass through blood-brain barrier (BBB) by a conventional administration into blood, for example, substances having a molecular weight of more than 1000. Especially, the present invention is more useful for peptides which hardly pass through BBB even by receptor-mediated transcytosis.

The present invention is also useful for substances which need to exhibit their effects selectively in the brain over a long period of time in order to increase the therapeutic effect thereof or to reduce the peripheral or systemic side effects, even though they can be transported and distributed in the brain without disturbance of BBB when they are administered by a conventional route such as oral or injection route.

For instance, antibiotic agents and anticancer agents are exemplified. In the treatment of cephalomeningitis, there has been desired an antibiotic agent which can be continuously distributed in cerebrospinal fluid. There has been desired an anticancer agent which can be distributed in a high concentration thereof and can exhibit the anticancer activity thereof selectively in the brain, especially within the lesion of brain tumor, over a long period of time.

The above active substances are different in the physicochemical properties thereof, but are all expected to be useful as a medicament for the treatment of cerebral diseases and the clinical effects thereof are expected to be enhanced by extended distribution and the long duration time of action in brain parenchyma or in medullary cavity.

Especially, it is therapeutically important that the neurotrophic factors are distributed and exhibit the activity thereof throughout brain parenchyma, and by administering the sustained-release preparation thereof prepared according to the present invention into brain parenchyma or CSF space, the unexpected effect which cannot be obtained by a conventional administration route is obtained. For instance, as is shown in Experiments disclosed hereinafter NGF (molecular weight: 13,250) showed excellent effects by the present invention, and as to BDNF, CNTF, EGF and FGF which are a high molecular peptide having a molecular weight similar to NGF, i.e. 13,250, 20,400, 6,400 and 13,000, respectively, the preparation thereof prepared according to the present invention are expected to show an excellent activity like NGF. Further, hippocampus factor has been reported to have the same activity as NGF to enhance the growth of cholinergic neurons in the cultured medial septum nucleus of embryonic rat brain [Ojika, Jpn. J. Psycopharmacology, 7, 447 - 451 (1987)].

The process for preparing the sustained-release preparation of the present invention is carried out under mild conditions, and does not require any organic solvent and heating, and hence, the present invention can be applied to all of the above active substances.

The active substances for the treatment of cerebral diseases used in the present invention may be prepared by any conventional methods such as extraction from living body, artificial synthesis and gene recombination technology,

The sustained-release preparation of the present invention can be prepared by incorporating the active substance into a biodegradable carrier. Such as collagen and gelatin.

Collagen used as a biodegradable carrier in the present invention is the major protein of the animal connective tissue, and is a safe substance having low antigenicity so that it is widely used in the medical field, for example, as surgical sutures. Atelocollagen may also be used, which is prepared by removing the telopeptide portion of the collagen molecule in order to reduce the antigenicity thereof for the purpose of increasing the safety thereof. Moreover, chemically modified collagen such as succinylated collagen and methylated collagen, may also be used in the present invention. Gelatin is an induced protein derived from collagen, and it has been accepted as a safe substance in clinical use with low antigenicity and it is an inexpensive, high molecular ampholyte and has sol-gel transformation property. Gelatin can also be chemically modified like collagen. In the present invention, collagen, gelatin and above mentioned derivatives thereof may be used alone, or in the form of a mixture thereof in a suitable ratio.

The active substances for the treatment of cerebral diseases and biodegradable carriers used in the present invention are desired to be pure, but the usually available ones may also be used. The active substances or biodegradable carriers on the market usually contain an appropriate amount of additives such as a stabilizer, or a buffering agent. For instance, a solution of collagen on the market generally contains a buffer of an inorganic or organic salt such as phosphate buffer, citrate buffer or acetate buffer.

The mixing ratio of the active substance and the biodegradable carrier is not necessarily specified, but the active substance may be mixed, for example, in the ratio of 0.1 - 50 % by weight based on the weight of the carrier.

The sustained-release preparation of the present invention can be prepared by the following processes.

An active substance for the treatment of cerebral diseases, or an aqueous solution thereof is mixed under stirring with a biodegradable carrier or an aqueous solution thereof. That is, the active substance can be incorporated into a carrier matrix by mixing the active substance with the carrier in the state of a solution. Then, this mixture is concentrated and/or dried. The methods for concentration and drying are not specified. For example, the mixture may be concentrated by allowing to stand at room temperature. As a drying method, air-dry, spray dry or lyophilization, are exemplified, wherein the most suitable drying rate should be determined taking into consideration the amount of water to be removed and the preparation form. Accordingly, the drying is carried out under conditions of the most suitable temperature and humidity.

The above procedures are usually carried out at room temperature or a temperature below thereof, for example, under cooling. For example, the mixing is usually carried out at a temperature of about 5 to 30°C, and drying by lyophilization is usually carried out at a temperature of about -50 to 0°C, and further, other drying such as air-dry or spray dry are usually carried out with keeping the temperature of the active substance below 40°C by controlling the temperature of the solution and the vessel thereof below room temperature so that the active substance is not substantially damaged.

Besides, the above processes can be carried out without any specific binding agent and without heating, and hence, they are suitable especially for the active substances which are unstable to heat.

It is desirable that the sustained-release preparation of the present invention comprises only an active substance for the treatment of cerebral diseases and a biodegradable carrier. When the sustained-release preparation of the present invention contains other components in addition to the active substance and the carrier, the release of the active substance is promoted by such other components, and hence, it is desirable to exclude these other components therefrom as much as possible. However, even though the commercially available active substances and biodegradable carriers contain other components, they may be used as they stand, unless they affect the sustained-release property of the preparation. Similarly, if necessary, the sustained-release preparation of the present invention may contain conventional pharmaceutically acceptable additives such as a stabilizer, preservative or local anesthetic, within the extent which does not affect on the sustained-release property of the preparation. Further, the sustained-release preparation of the present invention may also contain an additive in order to control the release rate of active substance to obtain the most suitable release behavior according to the properties of the drug to be used and the subject to be treated.

The product thus obtained is properly processed so as to make it fit to the usage. For instance, dried product is pulverized and the resulting powder is compressed with a mold to form a suitable shaped product. Alternatively, a mixed solution of an active substance and a biodegradable carrier is added into a mold, and the mixture is concentrated or dried by air-dry or lyophilization, and then compressed to form the desired shaped product. Moreover, a gel mixture of an active substance and a carrier is formed into the desired shaped product by a conventional extrusion molding or injection molding and then dried.

The shaped form of the preparation may take any form, such as spherical, hemispherical, needle-like, cylindrical, button-like, filmy, disc-like or powdery, so as to adapt it to the part to be applied for administration into the brain parenchyma or cerebral CSF space. For example, the sustained-release preparation of the present invention can be implanted or inserted into the brain by using a device, for example, a catheter, or can be maintained in the brain during the operation.

When the sustained-release preparation of the present invention is in a needle-like shape or a cylindrical shape, the size thereof is, for example, about 0.1 - 10 mm in diameter and about 1 - 50 mm in length, preferably, about 0.5 - 2 mm in diameter and about 5 - 15 mm in length.

The dose of the active substance for the treatment of cerebral diseases used in the present invention may vary depending on the kinds of the active substance but the preparation of the invention is usually administered once or more a month at a dose (as the active substance) of 1 ng to 1 g/day in adult human.

The present invention is illustrated in more detail by the following Examples and Experiments, but should not be construed to be limited thereto.

Example 1

A 0.2 % aqueous solution (5 ml) of human serum albumin (HSA) containing NGF (2 mg) extracted and purified from mouse submaxillary gland by the method of W.C. Mobley et al. [Cf: Biochemistry 15, 5543 (1976)] and a 2 % aqueous solution (5 g) of atelocollagen are uniformly mixed, and lyophilized. Thereto is added a small amount of distilled water to swell, and thereto is added additional distilled water to adjust the total amount to 400 mg. The mixture is well kneaded to give a homogeneous mixture. The mixture is charged in a disposable syringe (capacity: 1 ml) and centrifugally deaerated at 10,000 G for 30 minutes. The mixture is extruded from the syringe and dried at 5°C under humidity of 75 % for 24 hours, and then, allowed to stand to dryness in a desiccator containing silica gel at room temperature for 24 hours. After drying in a desiccator, the product is cut to give bar-like sustained-release preparations containing 20 μg/rod of NGF (0.45 mm in diameter, 4 mm in length).

Example 2

A 50 mM aqueous solution (10 ml) of sodium acetate containing NGF (100 μg) extracted from mouse submaxillary gland and a 2 % aqueous solution (10 g) of atelocollagen containing dipotassium hydrogen phosphate (1.4 %) and potassium dihydrogen phosphate (0.3 %) are uniformly mixed with stirring, and lyophilized. After lyophilization, the product is pulverized at a low temperature under liquid nitrogen. The resulting pulverized product (20 mg) is added into a mold and subjected to compression molding to give a disc-shaped sustained-release preparation (6 mm in diameter).

Example 3

To a 10 % aqueous solution (20 ml) of gelatin is added tris-glycine solution (5 ml) containing interferonα (2 x 10^6 U/ml), and a part thereof is poured into a hemispherical mold (3 mm in diameter) and lyophilized. The lyophilized product in the mold is subjected to compression molding to give a hemispherical sustained-release preparation.

Example 4

A 1% aqueous solution of gelatin obtained by heat-denaturalization of atelocollagen and a 1 % aqueous solution of atelocollagen are mixed in the ratio of 2 : 8 by weight to give an aqueous mixture thereof (5 ml). Thereto is added an aqueous solution of interferon-α (1 x 10^7 U/ml) (1 ml) and the mixture is uniformly mixed. This aqueous mixed solution is put into a plate, and allowed to stand at room temperature for 48 hours to give a filmy sustained-release preparation.

Experiment 1

Method:

The sustained-release preparation of NGF of the present invention obtained in Example 1 was stereotaxically inserted into the left dorsal hippocampus of a Mongolian gerbil. In the control group, placebo preparation containing only atelocollagen without NGF was used instead of the sustained-release preparation of the present invention in the same way. One day after administration, the Mongolian gerbil was anesthetized with ketamine hydrochloride and the bilateral common carotid artery was occluded for 10 minutes, and then, opened again. Four days later, the Mongolian gerbil was sacrificed by decapitation. The brain was removed and was fixed in a mixture of ethanol and acetic acid (95 : 5), and mounted in paraffin. Therefrom, the coronal paraffin section with thickness of 6 $\mu$m was prepared and subjected to hematoxylin and eosin stain. The CAI pyramidal cell layer of the dorsal hippocampus was subdivided into pieces of 200 $\mu$m width. The number of survived cells in each division was counted. Further, the tissues of various parts of the brain were taken out one day and five days after inserting the preparation, and the concentration of NGF in each brain tissue was determined by enzyme immunoassay (EIA).

Results:

In the Mongolian gerbil of the control group, the disintegration of a lot of pyramidal cells was observed either in the inserted hippocampus (the left side) or in the opposite hippocampus (the right side). On the other hand, in the Mongolian gerbil of the treated group of the NGF sustained-release preparation of the present invention, the disintegration of pyramidal cells was little observed not only in the inserted hippocampus but also in the opposite hippocampus. That is, the significant increase in the number of surviving cells in the Mongolian gerbil of the NGF sustained-release preparation treated group was observed in the region of 200 - 400 $\mu$m outside from the inserted point, and throughout the opposite hippocampus (Cf: Fig. 1) in comparison with the control group.

The results of determination of NGF concentration in the various parts of brain are shown in Table 1. In the gerbil of the NGF sustained-release preparation treated group, the concentration of NGF in the inserted part was higher than that in the opposite part, and on the whole, the concentration of NGF in the treated group was higher than that in the control group. The part wherein the concentration of NGF was the highest was the striatum, and the next was the occipital part of cerebral cortex. In the NGF sustained-release preparation treated group, the concentration of NGF was higher than that of the control group even five days after administration, suggesting that the NGF sustained-release preparation of the present invention has sustained-release property.

**Table 1** Concentration of NGF (ng/g) in various brain regions of Mongolian gerbil

| Part of the brain | | Test preparation NGF sustained-release preparation (20 μg) | | Placebo preparation | |
|---|---|---|---|---|---|
| | | 1 day | 5 days | 1 day | 5 days |
| Frontal part of cerebral cortex | Inserted side | 21.4 | 7.0 | 5.2 | 4.2 |
| | Opposite side | 12.0 | 3.8 | 4.5 | 1.7 |
| Striatum | Inserted side | 176.9 | 15.8 | – | 10.4 |
| | Opposite side | 126.8 | 8.4 | – | 1.9 |
| Occipital part of cerebral cortex | Inserted side | 137.8 | 21.3 | 17.4 | 3.8 |
| | Opposite side | 74.5 | 12.9 | 4.1 | 3.8 |
| Cerebellum | Inserted side | 12.4 | 3.0 | 2.5 | 1.5 |
| | Opposite side | 12.2 | 2.3 | 2.0 | 2.1 |

## Experiment 2

In the above Experiment 1, it became apparent that NGF is well distributed and the release thereof is well sustained in the various parts of brain by inserting the sustained-release preparation thereof of the present invention containing NGF (20 μg) into the hippocampus.

Further, in order to examine the properties of the present preparation in more detail, the concentration of NGF in six parts of brain including hippocampus which was not examined in Experiment 1, striatum, thalamus, cerebellum, and both the frontal part and the occipital part of cerebral cortex were determined at various period of time after administration such as 6 hours, 1 day, 3 days and 5 days. Besides, there were some control groups examined as reference, such as the placebo group (test group No. II) wherein a placebo preparation without NGF was inserted into the hippocampus, the NGF injected group (test group No. III) wherein NGF (20 μg) was directly injected into the hippocampus, the saline injected group (test group No. IV) wherein physiological saline was injected into the hippocampus, the NGF i.v. injected group (test group No. V) wherein NGF (20 μg) was intravenously injected, and the saline i.v. injected group (test group No. VI) wherein physiological saline was intravenously injected. The concentrations of NGF in the above various parts of brain of the Mongolian gerbil in the NGF sustained-release preparation of the present invention treated group (test group No. I) were compared with those of the Mongolian gerbil in the above control groups. The experiment was carried out in each group of one Mongolian gerbil, and the concentrations of NGF in the various parts of brain of a normal Mongolian gerbil (test group No. VII) were also determined for reference.

Method:

The NGF sustained-release preparation prepared in Experiment 1 was stereotaxically inserted into the left dorsal hippocampus of Mongolian gerbil. The preparation without NGF was inserted into the Mongolian gerbil in the placebo group. NGF (20 μg) was directly injected into the hippocampus tissue in the NGF injected group. NGF (20 μg) was intravenously injected in the NGF i.v. injected group. Physiological saline was intravenously injected in the saline i.v. injected group. The Mongolian gerbils were anesthetized with ketamine hydrochloride one day after administration or insertion. The bilateral common carotid artery was occluded for 10 minutes and then opened again, and the Mongolian gerbils were killed with decapitation at various period of time such as 1 hour (only in the i.v. injected groups), 6 hours, 1 day, 3 days and 5 days after administration. The tissues of the various parts of brain were taken out, and the concentrations of NGF therein were determined by EIA method.

Results:

The results of determination of the NGF concentration in the tissues of the various parts of brain are shown in Tables 2, 3 and 4. In the Mongolian gerbil inserted with the NGF sustained-release preparation group (I), the concentration of NGF in the inserted side was about 20 - 1000 times as high as that of the normal Mongolian gerbil, and NGF was distributed almost throughout the brain. Especially, the concentrations of NGF in the hippocampus, the thalamus and the occipital part of cerebral cortex were high. Besides, even five days after administration, the concentration of NGF was more than 2 ng/g, and the high concentration thereof is sustained. Further, the concentrations of NGF in the opposite side were generally lower than those of the corresponding parts on the inserted side, but the same tendency as in the inserted side and the distribution of NGF throughout she brain were also observed. On the other hand, in the NGF injected group (III) wherein NGF (20 μg) was directly inserted into the hippocampus, the highest concentration of NGF is observed 6 hours after administration, but the concentration thereof at five days after administration was decreased to almost the same level as that of the normal Mongolian gerbil, and the sustained-release effect as observed in the NGF sustained-release preparation group was not observed. Further, the concentrations of NGF in the opposite side of brain in the
NGF injected group were not as high as those in the NGF sustained-release preparation group. In the NGF i.v. injected group, the concentration of NGF in the brain even at 1 hours after administration was less than 1 ng/g and almost the same as that of the normal Mongolian gerbil.

Table 2  NGF concentration (ng/g) in various brain regions of Mongolian gerbil

| Test Group | | (I): NGF (20 $\mu$g) sustained-release. prep. inserted into hippocampus | | | | (II): Placebo prep. inserted into hippocampus | |
|---|---|---|---|---|---|---|---|
| Time after administration | | 6 hours | 1 day | 3 days | 5 days | 6 hours | 5 days |
| Frontal part of cerebral cortex | Inserted side (left) | 7.79 | 6.96 | 11.05 | 9.18 | 1.30 | 0.27 |
| | Opposite side (right) | 4.13 | 13.96 | 7.12 | 2.13 | 0.88 | 0.63 |
| Striatum | Inserted side (left) | 3.98 | 29.22 | 22.85 | 2.50 | 0.78 | 0.36 |
| | Opposite side (right) | 3.84 | 10.87 | 11.52 | 2.61 | 1.27 | 0.94 |
| Hippocampus | Inserted side (left) | 31.76 | 61.48 | 72.74 | 42.86 | 1.06 | 0.57 |
| | Opposite side (right) | 21.08 | 28.30 | 17.59 | 9.13 | 2.52 | 0.25 |
| Thalamus | Inserted side (left) | 131.55 | — | 17.67 | 9.44 | 0.15 | 0.14 |
| | Opposite side (right) | 4.57 | 15.10 | 8.24 | 11.02 | 0.94 | 0.34 |
| Occipital part of cerebral cortex | Inserted side (left) | 41.36 | — | 59.70 | 17.38 | 1.76 | 1.09 |
| | Opposite side (right) | 5.71 | 23.06 | 6.37 | 3.25 | 1.01 | 0.36 |
| Cerebellum | Inserted side (left) | 4.57 | 38.83 | 7.05 | 2.25 | 2.25 | 0.14 |
| | Opposite side (right) | 2.07 | 41.92 | 5.38 | 1.81 | 0.75 | 0.09 |

Table 3 NGF concentration (ng/g) in various brain regions of Mongolian gerbil

| Test Group | | (III): NGF (20 μg) injected into hippocampus | | | | (IV): Saline injected into hippocampus |
|---|---|---|---|---|---|---|
| Time after administration | | 6 hours | 1 day | 3 days | 5 days | 6 hours |
| Frontal part of cerebral cortex | Inserted side (left) | 1.12 | 2.26 | 0.43 | 0.52 | 0.22 |
| | Opposite side (right) | 0.54 | 2.77 | 0.16 | 0.29 | 0.15 |
| Striatum | Inserted side (left) | 1.14 | 41.85 | 0.33 | 0.30 | 0.10 |
| | Opposite side (right) | 0.97 | 2.02 | 0.21 | 1.21 | 0.20 |
| Hippocampus | Inserted side (left) | 24.59 | 9.00 | 0.46 | 1.24 | 0.43 |
| | Opposite side (right) | 2.18 | 3.55 | 0.16 | 0.90 | 0.14 |
| Thalamus | Inserted side (left) | 26.82 | 5.36 | 0.16 | 0.07 | 0.09 |
| | Opposite side (right) | 1.07 | 2.92 | 0.43 | 0.11 | 0.08 |
| Occipital part of cerebral cortex | Inserted side (left) | 6.51 | 5.55 | 0.32 | 0.16 | 0.34 |
| | Opposite side (right) | 5.01 | 1.65 | 0.28 | 0.39 | 0.47 |
| Cerebellum | Inserted side (left) | 2.80 | 8.38 | 0.66 | 0.13 | 0.07 |
| | Opposite side (right) | 1.68 | 8.83 | 0.15 | 0.06 | 0.06 |

EP 0 412 554 B1

Table 4 NGF concentration (ng/g) in various brain regions of Mongolian gerbil

| Test Group | | (V): NGF (20 µg) i.v. injected | (VI): Saline i.v. injected | (VII): Normal Mongolian gerbil |
|---|---|---|---|---|
| Time after administration | | 1 hour | 1 hour | – |
| Frontal part of cerebral cortex | Inserted side (left) | 0.18 | 0.10 | 0.09 |
| | Opposite side (right) | 0.14 | 0.05 | 0.08 |
| Striatum | Inserted side (left) | 0.15 | 0.08 | 0.18 |
| | Opposite side (right) | 0.11 | 0.15 | 0.05 |
| Hippocampus | Inserted side (left) | 0.20 | 0.12 | 0.16 |
| | Opposite side (right) | 0.24 | 0.28 | 0.20 |
| Thalamus | Inserted side (left) | 0.30 | 0.05 | 0.13 |
| | Opposite side (right) | 0.55 | 0.13 | 0.14 |
| Occipital part of cerebral cortex | Inserted side (left) | 0.34 | 0.20 | 0.40 |
| | Opposite side (right) | 0.31 | 0.15 | 0.13 |
| Cerebellum | Inserted side (left) | 0.47 | <0.05 | <0.04 |
| | Opposite side (right) | 0.49 | <0.03 | 0.04 |

Example 5

A 0.2 % aqueous solution (5 ml) of human serum albumin (HSA) containing mouse epidermal growth factor (300 µg) (m-EGF, Collaborative Research Inc., USA) and a 2 % aqueous solution (5 g) of

EP 0 412 554 B1

atelocollagen are uniformly mixed, and lyophilized. Thereto is added a small amount of distilled water to swell, and thereto is added additional distilled water to adjust the total amount to 400 mg. The mixture is well kneaded to give a homogeneous mixture. The mixture is charged in a disposable syringe (capacity: 1 ml) and centrifugally deaerated at 10,000 G for 30 minutes. The mixture is extruded from the syringe and dried at 5°C under humidity of 75 % for 24 hours, and then, allowed to stand to dryness in a desiccator containing silica gel at room temperature for 24 hours. After drying in a desiccator, the product is cut to give bar-like sustained-release preparations containing 20 µg/rod of m-EGF (0.9 mm in diameter, 9 mm in length).

Experiment 3: In vitro release test of EGF

Sample obtained in Example 5 was placed in 40 ml of phosphate-buffered saline solution containing HSA (5 mg/ml), pH 7.4 and incubated at 37°C. The amount of m-EGF released into the solution from the sample was measured by radioimmunoassay (Amersham International plc., UK), and accumulated amount of m-EGF released was determined with a lapse of time. The result is shown in Fig. 2.

Example 6

An aqueous solution containing 5 µg/ml of bovine basic fibroblast growth factor (b-FGF, R & D SYSTEMS, INC.) (2 ml) and a 2 % aqueous solution (50 g) of atelocollagen are uniformly mixed, and lyophilized. Thereto is added a small amount of distilled water to swell, and thereto is added additional distilled water to adjust the total amount to 3,400 mg. The mixture is well kneaded to give a homogeneous mixture. The mixture is charged in a disposable syringe (capacity: 5 ml) and centrifugally deaerated at 10,000 G for 60 minutes. The mixture is extruded from the syringe and dried at 5°C under humidity of 75 % for 48 hours, and then, allowed to stand to dryness in a desiccator containing silica gel at 5°C for 72 hours. After drying in a desiccator, the product is cut to give bar-like sustained-release preparations containing 10 ng/rod of b-FGF (1 mm in diameter, 9 mm in length).

Effects of the present preparation:

Since biologically active peptides with high molecular weight such as NGF cannot easily diffuse in the brain tissue, it has been expected that the effects thereof would be limited within the part administered. However, as is shown in the Experiments above, the NGF administered into the one side of the dorsal hippocampus in the form of a sustained-release preparation according to the present invention is maintained in the tissues over a long period of time, and unexpectedly, the high concentration of NGF is detected even in the opposite side of the insertion of the present preparation. This fact indicates that the diffuse and continuous distribution of brain peptides throughout the brain is made possible by the preparation of the present invention. Moreover, as is apparent from the results of Experiment 1, the present preparation has an great effect which cannot be obtained by any conventional preparation or by any conventional administration method.

According to the present invention, a practical and effective method for the treatment of cerebral diseases and for primary or metastatic cerebral tumor can be expected.

**Claims**

**Claims for the following Contracting States : AT, BE, DE, DK, FR, GB, IT, NL, SE**

1. Use of a brain peptide, a derivative thereof, an antibody, an agonist or an antagonist thereof as an active substance incorporated into a biodegradable carrier selected from the group consisting of collagen, gelatin or a mixture thereof for the preparation of a sustained-release preparation for the treatment of cerebral diseases whereby the treatment achieves diffusion and continuous distribution of the active substance throughout the brain.

2. The use according to claim 1, wherein said treatment is by administering said preparation into the brain.

3. The use according to claims 1 or 2, wherein said sustained-release preparation is implanted within the brain.

14

EP 0 412 554 B1

4. The use according to claims 1 or 2, wherein said sustained-release preparation is inserted within the brain.

5. The use according to any one of claims 1 to 4, wherein said active substance is an antibiotic agent or an anticancer agent.

6. The use according to anyone of claims 1 to 4, wherein said brain peptide is a neurotrophic factor, a cell growth factor, a neurotransmitter-related compound, an immuno-stimulator or a tumor necrosis factor.

7. The use according to claim 6, wherein said neurotrophic factor is nerve growth factor (NGF), brain derived neurotrophic factor (BDNF), ciliary neurotrophic factor (CNTF), NT-3 or hippocampus-derived neurotrophic factor.

8. The use according to claim 7, wherein said hippocampus-derived neurotrophic factor is hippocampus factor.

9. The use according to claim 6, wherein said cell according to claim 7, wherein said cell growth factor is epidermal growth factor (EGF) or fibroblast growth factor (FGF).

10. The use according to claim 6, wherein said neurotransmitter-related compound is cholecystokinin (CCK) or vasopressin.

11. The use according to claim 6, wherein said immuno-stimulator is interleukin or interferon.

12. The use according anyone of to claim 1 to 12, wherein said biodegradable carrier is collagen.

13. The use according to claim 12, wherein said collagen is atelocollagen.

14. The use according to any one of claims 1 to 13, wherein said sustained-release preparation is a formed solid preparation.

15. The use according to any one of claims 1-4 and 6-14, wherein said substance for the treatment of cerebral diseases is a substance which is not substantially transferred through the blood-brain barrier.

16. The use according to claim 6, wherein the neurotrophic factor is nerve growth factor.

17. The use according to claim 6, wherein the neurotrophic factor is brain derived neurotrophic factor.

18. The use according to claim 6, wherein the neurotrophic factor is ciliary neurotrophic factor.

19. The use according to claim 6, wherein the immuno-stimulator is interferon.

**Claims for the following Contracting States : ES, GR**

1. A method for the preparation of a sustained-release preparation for the treatment of cerebral diseases whereby the treatment achieves diffusion and continuous distribution of the active substance throughout the brain comprising incorporating a brain peptide, a derivative thereof, an antibody, an agonist or an antagonist thereof as an active substance into a biodegradable carrier selected from the group consisting of collagen, gelatin or a mixture thereof.

2. The-method according to claim 1, wherein said treatment is by administering said preparation into the brain.

3. The method according to claims 1 or 2, wherein said sustained-release preparation is to be implanted within the brain.

4. The method according to claims 1 or 2, wherein said sustained-release preparation is to be inserted within the brain.

15

EP 0 412 554 B1

5. The method according to any one of claims 1 to 4, wherein said active substance in an antibiotic agent or an anticancer agent.

6. The method according to any one of claims 1 to 4, wherein said brain peptide is a neurotrophic factor, a call growth factor, a neurotransmitter-related compound, an immuno-stimulator or a tumor necrosis factor.

7. The method according to claim 6, wherein said neurotrophic factor is nerve growth factor (NGF), brain derived neurotrophic factor (BDNF), ciliary neurotrophic factor (CNTF), NT-3 or hippocampus-derived neurotrophic factor.

8. The method according to claim 7, wherein said hippocampus-derived neurotrophic factor is hippocampus factor.

9. The method according to claim 6, wherein said cell growth factor in epidermal growth factor (EGF) or fibroblast growth factor (FGF).

10. The method according to claim 6, wherein said neurotransmitter-related compound in cholecystokinin (CCK) or vasopressin.

11. The method according to claim 6, wherein said immuno-stimulator is interleukin or interferon.

12. The method according to any one of claims 1 to 12, wherein said biodegradable carrier is collagen.

13. The method according to claim 12, wherein said collagen is atelocollagen.

14. The method according to any one of claims 1 to 13, wherein said sustained-release preparation is a formed solid preparation.

15. The method according to any one of claims 1-4 and 6-14, wherein said substance for the treatment of cerebral diseases is a substance which is not substantially transferred through the blood-brain barrier.

16. The method according to claim 6, wherein the neurotrophic factor is nerve growth factor.

17. The method according to claim 6, wherein the neurotrophic factor is brain derived neurotrophic factor.

18. The method according to claim 6, wherein the neurotrophic factor is ciliary neurotrophic factor.

19. The method according to claim 6, wherein the immuno-stimulator is interferon.

**Patentansprüche**
**Patentansprüche für folgende Vertragsstaaten : AT, BE, DE, DK, FR, GB, IT, NL, SE**

1. Verwendung eines Hirnpeptids, eines Derivats davon, eines Antikörpers, eines Agonisten oder eines Antagonisten davon als Wirkstoff, der einem biologisch abbaubaren Träger aus der Gruppe Collagen, Gelatine oder ein Gemisch davon einverleibt ist, zur Herstellung einer Zubereitung mit verzögerter Abgabe zur Behandlung von zerebralen Erkrankungen, wobei die Behandlung eine Diffusion und kontinuierliche Verteilung des Wirkstoffs im gesamten Gehirn erreicht.

2. Verwendung nach Anspruch 1, wobei die Behandlung durch Verabreichung der Zubereitung in das Gehirn erfolgt.

3. Verwendung nach Anspruch 1 oder 2, wobei die Zubereitung mit verzögerter Abgabe in das Gehirn implantiert wird.

4. Verwendung nach Anspruch 1 oder 2, wobei die Zubereitung mit verzögerter Abgabe in das Gehirn inseriert wird.

16

**5.** Verwendung nach einem der Ansprüche 1 bis 4, wobei es sich beim Wirkstoff um ein antibiotisches Mittel oder um ein Antikrebsmittel handelt.

**6.** Verwendung nach einem der Ansprüche 1 bis 4, wobei es sich beim Hirnpeptid um einen neurotropen Faktor, einen Zellwachstumsfaktor, eine neurotransmitterverwandte Verbindung, einen Immunostimulator oder einen Tumornekrosefaktor handelt.

**7.** Verwendung nach Anspruch 6, wobei es sich beim neurotropen Faktor um Nervenwachstumsfaktor (NGF), vom Gehirn abgeleiteten neurotropen Faktor (BDNF), ziliaren neurotropen Faktor (CNTF), NT-3 oder vom Hippocampus abgeleiteten neurotropen Faktor handelt.

**8.** Verwendung nach Anspruch 7, wobei es sich beim vom Hippocampus abgeleiteten neurotropen Faktor um Hippocampus-Faktor handelt.

**9.** Verwendung nach Anspruch 6, wobei es sich beim Zellwachstumsfaktor um Epidermis-Wachstumsfaktor (EGF) oder Fibroblasten-Wachstumsfaktor (FGF) handelt.

**10.** Verwendung nach Anspruch 6, wobei es sich bei der neurotransmitterverwandten Verbindung um Cholecystokinin (CCK) oder Vasopressin handelt.

**11.** Verwendung nach Anspruch 6, wobei es sich beim Immunostimulator um Interleukin oder Interferon handelt.

**12.** Verwendung nach einem der Ansprüche 1 bis 11, wobei es sich beim biologisch abbaubaren Träger um Collagen handelt.

**13.** Verwendung nach Anspruch 12, wobei es sich beim Collagen um Atelocollagen handelt.

**14.** Verwendung nach einem der Ansprüche 1 bis 13, wobei es sich bei der Zubereitung mit verzögerter Abgabe um eine geformte feste Zubereitung handelt.

**15.** Verwendung nach einem der Ansprüche 1 bis 4 und 6 bis 14, wobei es sich beim Wirkstoff zur Behandlung von Gehirnkrankheiten um einen Wirkstoff handelt, der im wesentlichen nicht durch die Blut-Hirn-Schranke hindurch übertragen wird.

**16.** Verwendung nach Anspruch 6, wobei es sich beim neurotropen Faktor um Nervenwachstumsfaktor handelt.

**17.** Verwendung nach Anspruch 6, wobei es sich beim neurotropen Faktor um vom Hirn abgeleiteten neurotropen Faktor handelt.

**18.** Verwendung nach Anspruch 6, wobei es sich beim neurotropen Faktor um ziliaren neurotropen Faktor handelt.

**19.** Verwendung nach Anspruch 6, wobei es sich beim Immunostimulator um Interferon handelt.

**Patentansprüche für folgende Vertragsstaaten : ES, GR**

**1.** Verfahren zur Herstellung einer Zubereitung mit verzögerter Abgabe zur Behandlung von zerebralen Erkrankungen, wobei die Behandlung eine Diffusion und kontinuierliche Verteilung des Wirkstoffs im gesamten Hirn erreicht, umfassend die Einverleibung eines Hirnpeptids, eines Derivats davon, eines Antikörpers, eines Agonisten oder eines Antagonisten davon als Wirkstoff in einen biologisch abbaubaren Träger aus der Gruppe Collagen, Gelatine oder ein Gemisch davon.

**2.** Verfahren nach Anspruch 1, wobei die Behandlung durch Verabreichung der Zubereitung in das Gehirn erfolgt.

17

3. Verfahren nach Anspruch 1 oder 2, wobei die Zubereitung mit verzögerter Abgabe in das Gehirn zu implantieren ist.

4. Verfahren nach Anspruch 1 oder 2, wobei die Zubereitung mit verzögerter Abgabe in das Gehirn zu inserieren ist.

5. Verfahren nach einem der Ansprüche 1 bis 4, wobei es sich beim Wirkstoff um ein antibiotisches Mittel oder um ein Antikrebsmittel handelt.

6. Verfahren nach einem der Ansprüche 1 bis 4, wobei es sich beim Hirnpeptid um einen neurotropen Faktor, einen Zellwachstumsfaktor, eine neurotransmitterverwandte Verbindung, einen Immunostimulator oder einen Tumornekrosefaktor handelt.

7. Verfahren nach Anspruch 6, wobei es sich beim neurotropen Faktor um Nervenwachstumsfaktor (NGF), vom Gehirn abgeleiteten neurotropen Faktor (BDNF), ziliaren neurotropen Faktor (CNTF), NT-3 oder vom Hippocampus abgeleiteten neurotropen Faktor handelt.

8. Verfahren nach Anspruch 7, wobei es sich beim vom Hippocampus abgeleiteten neurotropen Faktor um Hippocampus-Faktor handelt.

9. Verfahren nach Anspruch 6, wobei es sich beim Zellwachstumsfaktor um Epidermis-Wachstumsfaktor (EGF) oder Fibroblasten-Wachstumsfaktor (FGF) handelt.

10. Verfahren nach Anspruch 6, wobei es sich bei der neurotransmitterverwandten Verbindung um Cholecystokinin (CCK) oder Vasopressin handelt.

11. Verfahren nach Anspruch 6, wobei es sich beim Immunostimulator um Interleukin oder Interferon handelt.

12. Verfahren nach einem der Ansprüche 1 bis 11, wobei es sich beim biologisch abbaubaren Träger um Collagen handelt.

13. Verfahren nach Anspruch 12, wobei es sich beim Collagen um Atelocollagen handelt.

14. Verfahren nach einem der Ansprüche 1 bis 13, wobei es sich bei der Zubereitung mit verzögerter Abgabe um eine geformte feste Zubereitung handelt.

15. Verfahren nach einem der Ansprüche 1 bis 4 und 6 bis 14, wobei es sich beim Wirkstoff zur Behandlung von Gehirnkrankheiten um einen Wirkstoff handelt, der im wesentlichen nicht durch die Blut-Hirn-Schranke übertragen wird.

16. Verfahren nach Anspruch 6, wobei es sich beim neurotropen Faktor um Nervenwachstumsfaktor handelt.

17. Verfahren nach Anspruch 6, wobei es sich beim neurotropen Faktor um vom Hirn abgeleiteten neurotropen Faktor handelt.

18. Verfahren nach Anspruch 6, wobei es sich beim neurotropen Faktor um ziliaren neurotropen Faktor handelt.

19. Verfahren nach Anspruch 6, wobei es sich beim Immunostimulator um Interferon handelt.

**Revendications**
**Revendications pour les Etats contractants suivants : AT, BE, DE, DK, FR, GB, IT, NL, SE**

1. Utilisation d'un peptide cérébral, d'un dérivé de celui-ci, d'un anticorps, d'un agoniste ou d'un antagoniste de celui-ci comme substance active incorporée dans un véhicule biodégradable choisi dans le groupe formé par le collagène, la gélatine ou un mélange de ceux-ci pour la préparation d'une préparation à libération prolongée dur le traitement des maladies cérébrales, le traitement réalisant la diffusion et la distribution continue de la substance active dans le cerveau.

2. Utilisation selon la revendication 1, dans laquelle ledit traitement est accompli par administration de ladite préparation dans le cerveau.

3. Utilisation selon la revendication 1 ou 2, dans laquelle ladite préparation à libération prolongée est implantée dans le cerveau.

4. Utilisation selon la revendication 1 ou 2, dans laquelle ladite préparation à libération prolongée est insérée dans le cerveau.

5. Utilisation selon l'une quelconque des revendications 1 à 4, dans laquelle ladite substance active est un agent antibiotique ou un agent anticancéreux.

6. Utilisation selon l'une quelconque des revendications 1 à 4, dans laquelle ledit peptide cérébral est un facteur neurotrophique, un facteur de croissance cellulaire, un composé apparenté à un neurotransmetteur, un immunostimulateur ou un facteur nécrosant les tumeurs.

7. Utilisation selon la revendication 6, dans laquelle ledit facteur neurotrophique est un facteur de croissance des nerfs (NGF), un facteur neurotrophique dérivé du cerveau (BDNF), un facteur neurotrophique ciliaire (CNTF), NT-3 ou un facteur neurotrophique dérivé de l'hippocampe.

8. Utilisation selon la revendication 7, dans laquelle ledit facteur neurotrophique dérivé de l'hippocampe est un facteur hippocampique.

9. Utilisation selon la revendication 6, dans laquelle ledit facteur de croissance cellulaire est un facteur de croissance épidermique (EGF) ou un facteur de croissance des fibroblastes (FGF).

10. Utilisation selon la revendication 6, dans laquelle ledit composé apparenté à un neurotansmetteur est la cholécystokinine (CCK) ou la vasopressine.

11. Utilisation selon la revendication 6, dans laquelle ledit immunostimulateur est une interleukine ou un interféron.

12. Utilisation selon l'une quelconque des revendications 1 à 11, dans laquelle ledit véhicule biodégradable est le collagène.

13. Utilisation selon la revendication 12, dans laquelle ledit collagène est l'atélocollagène.

14. Utilisation selon l'une quelconque des revendications 1 à 13, dans laquelle ladite préparation à libération prolongée est une préparation solide façonnée.

15. Utilisation selon l'une quelconque des revendications 1 à 4 et 6 à 14, dans laquelle ladite substance pour le traitement des maladies cérébrales est une substance qui n'est pas sensiblement transférée à travers la barrière hématoencéphalique.

16. Utilisation selon la revendication 6, dans laquelle le facteur neurotrophique est un facteur de croissance des nerfs.

17. Utilisation selon la revendication 6, dans laquelle le facteur neurotrophique est un facteur neurotrophique dérivé du cerveau.

18. Utilisation selon la revendication 6, dans laquelle le facteur neurotrophique est un facteur neurotrophique ciliaire.

19. Utilisation selon la revendication 6, dans laquelle l'immunostimulateur est un interféron.

**EP 0 412 554 B1**

**Revendications pour les Etats contractants suivants : ES GR**

1. Procédé de préparation d'une préparation à libération prolongée pour le traitement des maladies cérébrales, le traitement réalisant la diffusion et la distribution continue de la substance active dans le cerveau, comprenant l'incorporation d'un peptide cérébral, d'un dérivé de celui-ci, d'un anticorps, d'un agoniste ou d'un antagoniste de celui-ci en tant que subatance active dans un véhicule biodégradable choisi dans le groupe formé par le collagène, la gélatine ou un mélange de ceux-ci.

2. Procédé selon la revendication 1, dans lequel ledit traitement est accompli par administration de ladite préparation dans le cerveau.

3. Procédé selon la revendication 1 ou 2, dans lequel ladite préparation à libération prolongée est destinée à être implantée dans le cerveau.

4. Procédé selon la revendication 1 ou 2, dans lequel ladite préparation à libération prolongée est destinée à être insérée dans le cerveau.

5. Procédé selon l'une quelconque des revendications 1 à 4, dans lequel ladite substance active est un agent antibiotique ou un agent anticancéreux.

6. Procédé selon l'une quelconque des revendications 1 à 4, dans lequel ledit peptide cérébral est un facteur neurotrophique, un facteur de croissance cellulaire, un composé apparenté à un neurotransmetteur, un immunostimulateur ou un facteur nécrosant les tumeurs.

7. Procédé selon la revendication 6, dans lequel ledit facteur neurotrophique est un facteur de croissance des nerfs (NGF), un facteur neurotrophique dérivé du cerveau (BDNF), un facteur neurotrophique ciliaire (CNTF), NT-3 ou un facteur neurotrophique dérivé de l'hippocampe.

8. Procédé selon la revendication 7, dans lequel ledit facteur neurotrophique dérivé de l'hippocampe est un facteur hippocampique.

9. Procédé selon la revendication 6, dans lequel ledit facteur de croissance cellulaire est un facteur de croissance épidermique (EGF) ou un facteur de croissance des fibroblastes (FGF).

10. Procédé selon la revendication 6, dans lequel ledit composé apparenté à un neurotransmetteur est la cholécystokinine (CCK) ou la vasopressine.

11. Procédé selon la revendication 6, dans lequel ledit immunostimulateur est une interleukine ou un interféron.

12. Procédé selon l'une quelconque des revendications 1 à 11, dans lequel ledit véhicule biodégradable est le collagène.

13. Procédé selon la revendicaion 12, dans lequel ledit collagène est l'atélocollagène.

14. Procédé selon l'une quelconque des revendications 1 à 13, dans lequel ladite préparation à libération prolongée est une préparation solide façonnée.

15. Procédé selon l'une quelconque des revendications 1 à 4 et 6 à 14, dans lequel ladite substance pour le traitement des maladies cérébrales est une substance qui n'est pas sensiblement transférée à travers la barrière hémato-encéphalique.

16. Procédé selon la revendication 6, dans lequel le facteur neurotrophique est un facteur de croissance des nerfs.

17. Procédé selon la revendication 6, dans lequel le facteur neurotrophique est un facteur neurotrophique dérivé du cerveau.

20

**18.** Procédé selon la revendication 6, dans lequel le facteur neurotrophique est un facteur neurotrophique ciliaire.

**19.** Procédé selon la revendication 6, dans lequel l'immunostimulateur est un interféron.

Fig. 1

NGF Sustained-Release Preparation Treated Group

Placebo Group

*: p < 0.05,   **: p < 0.01

EP 0 412 554 B1

Fig. 2

RELEASE PROFILE OF m−EGF

EP 0 412 554 B1